# EUROPEAN PATENT APPLICATION

(11) **EP 2 957 268 A1**
(43) Date of publication of application: **23.12.2015**
(21) Application number: 14172817.0
(22) Date of filing: 17.06.2014
(51) Int. Cl.: A61F 5/445, A61F 5/44

(54) **An ostomy pouching system**

(71) Applicant: University of Limerick, Co. Limerick Limerick (IE)
(72) Inventor: Hunt, Rhona, Roscommon, Co. Roscommon (IE); Kelleher, Kevin, Bailieborough, Co. Cavan (IE); Cavanagh, Leon Michael, Loughrea, Co. Galway (IE)
(74) Representative: Purdy, Hugh Barry

(57) **Abstract**

An ostomy pouching system comprises a fixing plate and a bag having an opening. The fixing plate comprises: a central hole for receipt of a stoma; a first major surface that in use faces a patient's body; a second major surface that in use faces away from the patient's body; means for attaching the fixing plate to a patient's body; and means for attaching the opening of the bag to the second major surface of the fixing plate surrounding the central hole. The central hole has a perimeter that is smaller than a perimeter of the stoma, and at least a portion of the first major surface that surrounds the central hole comprises a resiliently deformable region capable of stretching to form a neck region that conforms to a sidewall of the stoma when the stoma protrudes through the central hole.

## Description

### Introduction

The invention relates to an ostomy pouching system, and a fixing plate for an ostomy pouching system.

### Background to the Invention

The terms ostomy and stoma are general descriptive terms that are often used interchangeably though they have different meanings. An ostomy refers to the surgically created opening in the body for the discharge of body wastes. A stoma is the actual end of the ureter or small or large bowel that can be seen protruding through the abdominal wall. The most common specific types of ostomies are: colostomy, where a portion of the colon or rectum is removed and the remaining colon is brought to the abdominal wall and generally protrudes through the wall to form a stoma; ileostomy, where a surgically created opening in the small intestine, usually at the end of the ileum, is brought through the abdominal wall to form a stoma; and a urostomy, where a either a section of the small bowel or at the beginning of the large intestine is surgically removed and relocated as a passageway for urine to pass from the kidneys to the outside of the body through a stoma.

Ostomy pouching systems are known and generally comprise a bag having an opening for collection of matter that is passed through the ostomy, and a fixing system for attaching the bag to the body such that the opening surrounds the stoma. A fixing plate for a conventional ostomy system is described in Figs I and II and comprises a rigid plate A having a body engaging surface B and a hole C, a second surface D having a fitting E for attaching the bag (not shown) to the fitting. The plate A has a central circular portion F surrounding the hole C having a number of concentric circles G marked thereon, and an external part H that carries an adhesive for attaching the plate A to the users body. Generally, the adhesive will be covered by removable film (not shown). In use, a user will enlarge the hole C by cutting along one of the concentric circles, until the hole is slightly larger than the size of their stoma. The film covering the adhesive is then removed before the plate A is placed over the stoma, and pressed against the skin such that the adhesive adheres to the skin surface. At this point, the plate will be adhered to the skin and the stoma will; be protruding through the hole in the plate. The bag is then attached to the plate by means of the fitting E and a similar fitting at the mount of the bag (not shown).

A problem with devices of this type is that the cutting of the hole generally results in a hole that is quite a bit larger than the stoma, meaning that the patient's skin will be exposed between the base of the stoma and the edges of the hole. This allows faeces to come into contact with the skin, resulting in chemical dermatitis of the skin surrounding the ostomy. A further problem with this type of device is that as the hole is cut manually, and as the material of the plate is quite rigid. This results in sharp edges being formed along the periphery of the hole, which is uncomfortable for the user and could potentially result in damage to the walls of the stoma. Additionally, the use of adhesive causes, over prolonged use, mechanical dermatitis to the skin surrounding the stoma. Furthermore, the requirement to cut a hole in the fixing plate results in a considerable amount of patient inconvenience.

It is an object of the invention to overcome at least one of the above-referenced problems. In one embodiment, it is an object of the invention to provide an ostomy pouching system that helps reduce or overcome the problem of chemical dermatitis. In another embodiment, it is an object of the invention to provide an ostomy pouching system that helps reduce or overcome the problems of chemical and mechanical dermatitis.

### Statements of Invention

Broadly, the invention provides an ostomy pouching system that in one embodiment provides a solution to the problem of chemical dermatitis. This embodiment employs a fixing plate for attaching an ostomy bag to a patient, in which the skin-facing side of the plate comprises a flexible, resiliently deformable material, having a central hole that is dimensioned to be smaller than the stoma. When the plate is pressed against the skin such that the stoma protrudes through the hole, the flexible resiliently deformable material surrounding the hole will stretch around the stoma to form a neck region that tightly conforms to a sidewall of the stoma. This results in the skin around the base of the stoma being completely covered, thus preventing body fluids coming into contact with the skin and thereby preventing chemical dermatitis. The ostomy pouching system may be provided in the form of a separate bag and fixing plate, where the bag and plate are preferably adapted for engagement, or in a one-piece form in which the plate is attached to or integrally formed with the bag.

The invention also provides a second embodiment that addresses the problem of mechanical dermatitis. This embodiment employs a belt to attach the fixing plate to the stoma, obviating the need for adhesive attachment of the fixing plate to the patient's skin. The belt comprises a hole that in use is aligned with the stoma, and a peripheral region that abuts the fixing plate. In one embodiment, the fixing plate comprises base and upper layers connected by an annular seal that surrounds the central hole such that an annular peripheral portions of the base layer and upper layer are independent of each other. This allows the fixing plate engage with the belt with the base layer disposed against one face of the belt and the upper layer disposed against an opposite face of the belt.

In a first aspect, the invention provides an ostomy pouching system comprising a fixing plate and a bag having an opening, the fixing plate comprising: a central hole for receipt of a stoma; a first major surface that in use faces a patient's body; and a second major surface that in use faces away from the patient's body; characterised in that the central hole typically has a perimeter that is smaller than a perimeter of the stoma, and at least a portion of the first major surface that surrounds the central hole comprises a resiliently deformable region capable of stretching to form a neck region that conforms to a sidewall of the stoma when the stoma protrudes through the central hole.

The ostomy pouching system of the invention addresses the problem of chemical dermatitis by forming a neck region of flexibly deformable material that conforms to the sidewall of the stoma, thereby preventing body fluid (for example, faeces) coming into contact with the patients skin surrounding the base of the stoma. In order to achieve this, the central hole should be smaller than the stoma, and the region of the first major surface of the fixing plate surrounding the hole should be sufficiently flexible and deformable to stretch around the stoma. Examples of suitable materials for forming the resiliently deformable region include silicone materials that have the required degree of flexibility and also are sufficiently tacky to inhibit movement of the first major surface against the patient's skin. A further advantage of the system of the invention is that the flexibly deformable material can easily conform to stomata that are of irregular shape, whereas the devices of the prior art provide a template for a circular hole to be cut out and do not make allowance for stomata that are irregularly shaped. Typically, the ostomy pouching system comprises a fixing plate and a separate bag. In this embodiment, the plate and/or the bag are adapted for inter-engagement such that the opening in the bag surrounds the central hole of the fixing plate. Various engagement means are envisaged, for example snap-fit engagement means, hook and loop type engagement means, and adhesive means provided on the fixing plate, the bag, or both. In one embodiment, the system comprises separate adhesive means that can be applied to the bag or fixing plate (or both) by the patient.

In another embodiment, the pouching system of the invention comprises the fixing plate attached to the bag, and ideally integrally formed with the bag.

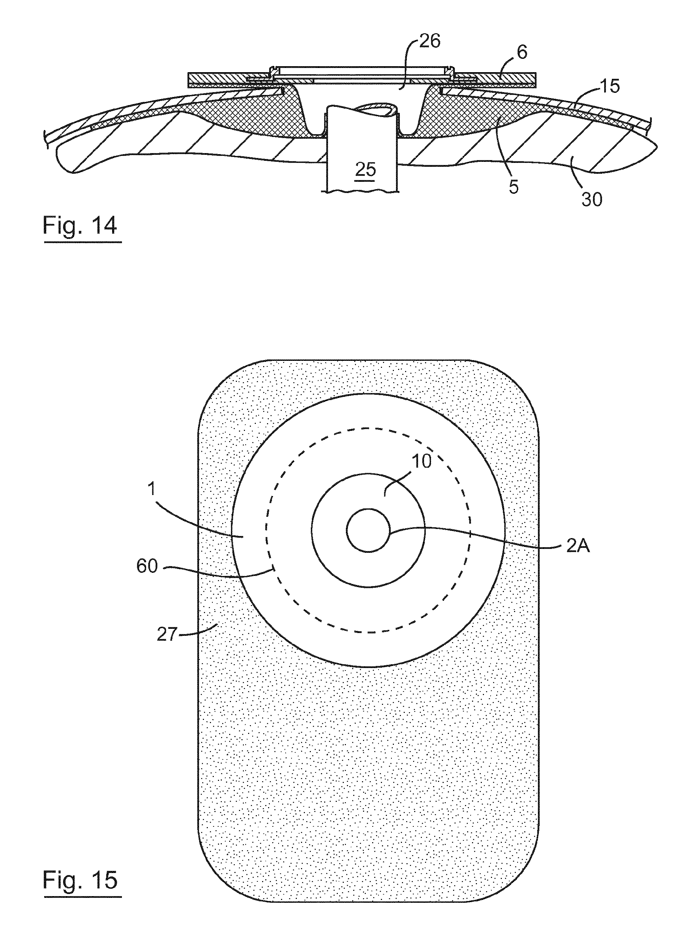

In one embodiment, the fixing plate has a convex face that in use abuts the patient's skin. This embodiment is described with reference to Fig. 14. The convex face has the effect of pressing down the abdomen around the patient's stoma, resulting in the stoma being raised. This enables the resiliently deformable region of the first major surface form a neck region on a sidewall of the stoma.

In one embodiment, the fixing plate comprises a layered structure having at least a base layer bearing the first major surface (and typically formed of a flexible tacky material) and an upper layer bearing the second major surface and formed of a material that is less flexible than the base layer. In this manner, the base layer is optimised for abutting the skin, and the upper layer can be optimised for other functions, for example attachment of the ostomy bag. Thus, in one embodiment, the upper layer bearing the second major surface may comprise a material suitable for adhering a layer of adhesive, and generally a backing paper to cover the adhesive prior to use. The upper layer bearing the second major surface may comprise a material suitable for reception of a layer of adhesive that may be applied to the bag. Thus, an adhesive layer may be applied to the bag, the second major surface of the fixing plate, or both. Alternatively, the second major surface of the fixing plate (or the part of the second major surface surrounding the central hole), or the bag (or a portion of the bag surrounding the bag opening) may comprise a material adapted to receive adhesive applied by the patient. A layer of polyurethane material (or a similar material) is suitable for receiving adhesive. In another embodiment, the upper layer bearing the second major surface may comprise a material that is suitable for attaching mechanical fixing means, for example a snap-fit attachment or a re-entrant slot type engagement means.

Typically, the base layer comprises an annular peripheral region surrounding the resiliently deformable region that is has a thickness greater than the thickness of the resiliently deformable region. This provides a base layer that is sufficiently robust to provide an anchoring means for the bag, and also has a thin, flexible, central portion adapted for engagement with the stoma as described above.

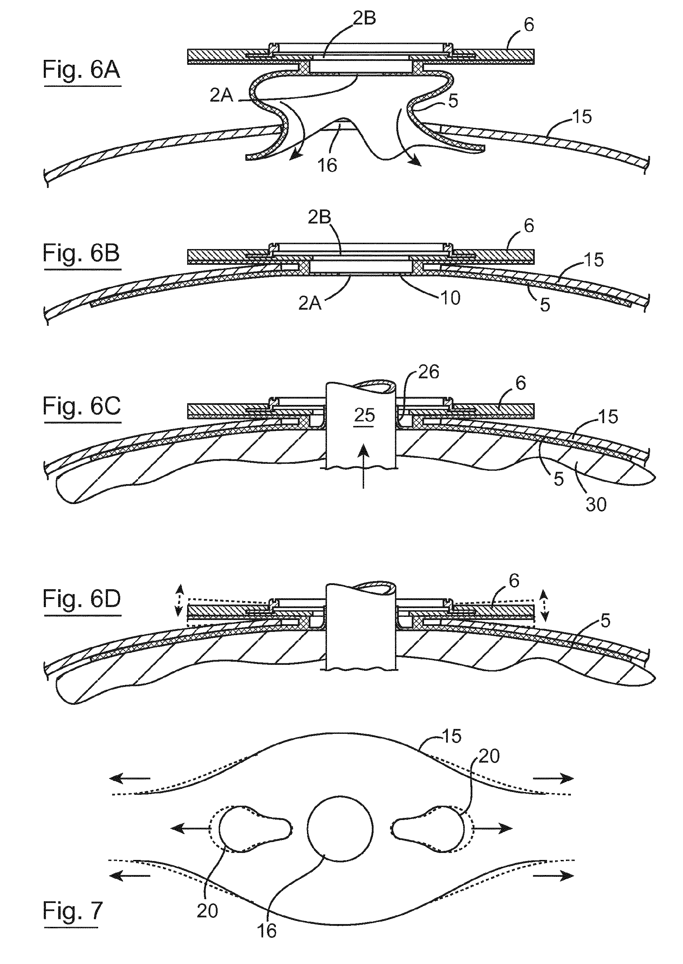

Preferably, the base layer and upper layer are connected by an annular seal that surrounds the central hole such that an annular peripheral portions of the base layer and upper layer are independent of each other (i.e. are not connected together). This is illustrated in Fig. 6D below, which shows that some movement of the upper layer is allowed without causing movement of the (stoma and skin engaging) base layer. This is important for the patient as it will give them confidence that accidental movement of the bag will not result in the stoma attachment being displaced. A further advantage of this feature is that it allows the fixing plate to be attached to a belt (as described below).

In one embodiment, the base layer has a convex face that in use abuts the patient's skin. This embodiment is described with reference to Fig. 14. The convex orientation of the base layer has the effect of pressing down the abdomen around the patient's stoma, resulting in the stoma being raised. This enables the resiliently deformable region of the base layer form a neck region on a sidewall of the stoma.

Preferably, the system comprises means for attaching the fixing plate to the patient's body. In one embodiment, the attachment means comprises adhesive. Suitably, the fixing plate comprises means for attachment to the patient's body, for example a layer of adhesive formed on the first major surface of the fixing plate. In another embodiment, the means for attaching the fixing plate to the patient's body comprises belt means having a central hole configured to receive the stoma and a surrounding surface adapted to abut the fixing plate. Use of a belt to attach/fix the fixing plate to the patient obviates the need to use adhesive attachment, and thereby overcomes the problem of mechanical dermatitis.

Typically, the belt comprises a further aperture located on each side of the central hole. This prevents the forces that are applied to the belt when tightening to be transmitted to the central hole (and thereby to the fixing plate), and provides for the forces to be channelled around the central hole. This is illustrated in Fig. 7.

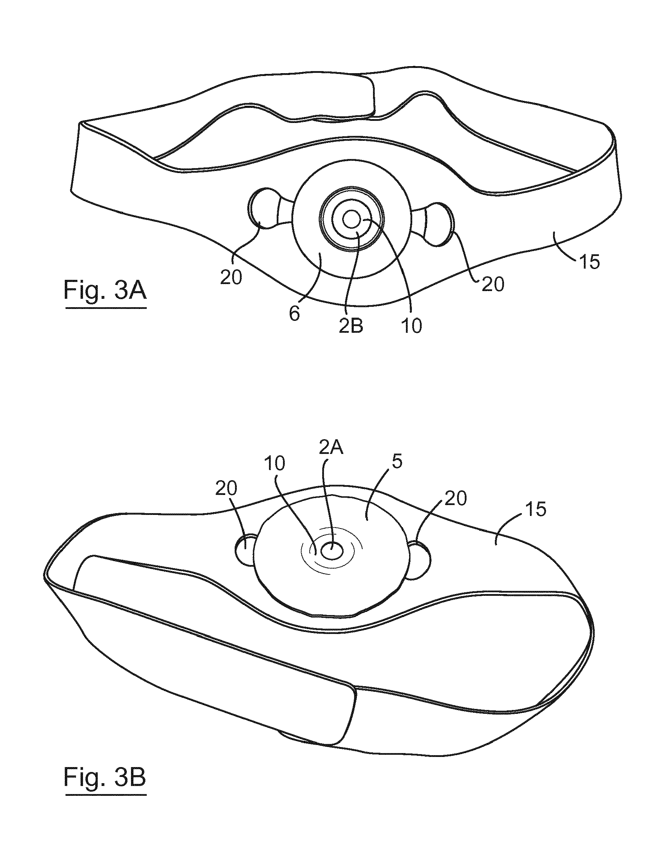

In one embodiment, the fixing plate is attached to the belt with the base layer disposed against one face of the belt and the upper layer disposed against an opposite face of the belt. This is best illustrated in Figs 3A and 3B and Figs 6A and 6B.

In another embodiment of the invention, the means for attaching the fixing plate to the patient's body comprises adhesive means disposed on the first major surface of the fixing plate. In this embodiment, a layer of adhesive, suitably covered by a backing paper, is disposed on at least part of the first major surface.

Suitably, system comprises means for attaching the fixing plate to a bag. Preferably, the attaching means comprises adhesive means or non-adhesive engagement means formed on the second major surface of the fixing plate or the bag or both. In one example, a layer of adhesive, suitably covered by a backing paper, is disposed on at least part of the second major surface. In another example, a snap fit attachment is provided on the second major surface, and a corresponding attachment is formed around the opening of the bag. Various forms of attachment means for secure attachment of the bag to the fixing means may be employed, the details of which will be known to the person skilled in the art.

In a related embodiment, the invention provides an ostomy pouching system comprising an ostomy bag having an opening for receipt of a stoma, wherein a periphery of the opening comprises a resiliently deformable material capable of stretching to form a neck region that in use conforms to a sidewall of the stoma when the stoma protrudes through the opening. Typically, the ostomy bag comprises an opening, and the periphery of the opening is formed by a layer of material adhered to the bag having an opening that is smaller than the opening of the bag. Suitably, the layer of material is disposed on an external surface of the bag and comprises the resiliently deformable material surrounding the opening and a peripheral part typically formed of a material that is more rigid than the resiliently deformable region. Suitably, the layer of material has an external surface that is suitable for adhering a layer of adhesive. In a related aspect, the invention also provides a fixing plate forming part of an ostomy pouching system of the invention, and comprising: a central hole for receipt of a stoma; a first major surface that in use faces a patient's body; a second major surface that in use faces away from the patient's body; optionally means for attaching the fixing plate to a patient's body; and optionally means for attaching the opening of the bag to the second major surface of the fixing plate surrounding the central hole, characterised in that the central hole has a perimeter that is smaller than a perimeter of the stoma, and at least a portion of the first major surface that surrounds the central hole comprises a resiliently deformable region capable of stretching to form a neck region that conforms to a sidewall of the stoma when the stoma protrudes through the central hole.

In one embodiment, the fixing plate comprises a layered structure having at least a base layer bearing the first major surface (and typically formed of a flexible tacky material) and an upper layer bearing the second major surface and formed of a material that is less flexible than the base layer. Thus, in one embodiment, the upper layer bearing the second major surface may comprise a material suitable for adhering a layer of adhesive, and generally a backing paper to cover the adhesive prior to use. In another embodiment, the upper layer bearing the second major surface may comprise a material that is suitable for attaching mechanical fixing means, for example a snap-fit attachment. A layer of polyurethane material (or a similar material) is suitable for these purposes.

Typically, the base layer comprises an annular peripheral region surrounding the resiliently deformable region that has a thickness greater than the thickness of the resiliently deformable region.

Preferably, the base layer and upper layer are connected by an annular seal that surrounds the central hole such that an annular peripheral portions of the base layer and upper layer are independent of each other (i.e. are not connected together).

In another embodiment of the invention, the means for attaching the fixing plate to the patient's body comprises adhesive means disposed on the first major surface of the fixing plate. In this embodiment, a layer of adhesive, suitably covered by a backing paper, is disposed on at least part of the first major surface.

Suitably, the means for attaching the fixing plate to the bag comprises adhesive means or non-adhesive engagement means formed on the second major surface of the fixing plate or the bag or both. In one example, a layer of adhesive, suitably covered by a backing paper, is disposed on at least part of the second major surface. In another example, a snap fit attachment is provided on the second major surface, and a corresponding attachment is formed around the opening of the bag. Various forms of attachments means for secure attachment of the bag to the fixing means may be employed, the details of which will be known to the person skilled in the art.

In one embodiment, the fixing plate is attached to, or integrally formed with, an ostomy bag.

The invention also provides a method of attaching an ostomy pouching system according to the invention to a patient having an ostomy, the method comprising the steps of: positioning the fixing plate against the body such that the stoma aligns with the central hole, pressing the fixing plate against the body such that the stoma is forced through the central hole and the resiliently deformable region of the first major surface stretches to form a neck region that conforms to a sidewall of the stoma, attaching the fixing plate to the patient's body, and attaching the opening of the bag to the second major surface of the fixing plate.

In another embodiment, the method of attaching an ostomy pouching system of the type having a layered fixing plate to a patient having an ostomy, the method comprising the steps of: attaching the fixing plate to the belt such that the base layer of the fixing plate is disposed against one face of the belt and the upper layer of the fixing plate is disposed against an opposite face of the belt, positioning the fixing plate and the belt against the body such that the stoma aligns with the central hole, pressing the fixing plate against the body such that the stoma is forced through the central hole and the resiliently deformable region of the first major surface stretches to form a neck region that conforms to a sidewall of the stoma, attaching the fixing plate to the patient's body using the belt, and attaching the opening of the bag to the second major surface of the fixing plate.

### Brief Description of the Figures

The invention will be more clearly understood form the following description of some embodiments thereof, given by way of example only, with reference to the accompanying figures in which:
Fig. I is perspective view of part of a known ostomy pouching system (shown without the bag-part of the pouching system) showing a body engaging face of the fixing plate;
Fig. II is a perspective view of the ostomy pouching system of Fig. I showing the opposite, bag-engaging face, of the fixing plate;
Fig. 1A is perspective view of a fixing plate forming part of an ostomy pouching system according to one embodiment of the invention showing the bag-engaging face of the fixing plate;
Fig. 1B is a perspective view of the fixing plate of Fig. 1 shown from the opposite (skin facing side);
Fig. 1C is a side elevation view of the fixing plate of Fig. 1A;
Figs 2A and 2B are perspective views of a belt forming part of one embodiment of the stoma pouching system of the invention;
Figs. 3A and 3B are perspective views of the fixing belt of Fig. 1A attached to the belt of Fig. 2A;
Fig. 4 is a perspective illustration of the assembly of Figs 3A and 3B in-situ on a patient, and showing the stoma protruding through the hole in the fixing plate;
Fig. 5 is side elevation, sectional, view of the fixing plate in-situ on a patient, and with an ostomy bag attached to the fixing plate;
Figs. 6A to 6C are a series of illustrations showing the attachment of the fixing plate to the belt (Figs. 6A and 6B), the attachment of the belt and plate assembly to the patient (Fig. 6C and 6D);
Fig. 7 is an illustration of part of the belt forming part of an ostomy pouching system on one embodiment of the invention;
Fig. 8 is an illustration of a fixing plate for an ostomy pouching system according to an alternative embodiment of the invention;
Fig. 9 is a side elevation, sectional, view of the fixing plate of Fig. 8 in-situ on a patient showing the stoma protruding through the hole in the fixing plate;
Fig. 10 is a side elevation, sectional, view of a fixing plate for an ostomy pouching system according to an alternative embodiment of the invention;
Fig. 11 is a side elevation, sectional, view of the fixing plate of Fig. 10 shown in-situ on a patient showing the stoma protruding through the hole in the fixing plate;
Fig. 12 is a perspective view of the fixing plate of Fig. 10 shown in-situ on a patient showing the stoma protruding through the hole in the fixing plate;
Fig. 13 is side elevation, sectional, view of the fixing plate of Fig. 10 in-situ on a patient, and with an ostomy bag attached to the fixing plate;
Fig. 14 is a side elevation view of a fixing plate according to an alternative embodiment of the invention and suitable for use with patients having a short stoma or a soft abdomen; and
Fig. 15 is an illustration of an ostomy pouching system according to an alternative embodiment of the invention in which the fixing plate and ostomy bag are integrally formed.

### Detailed Description of the Invention

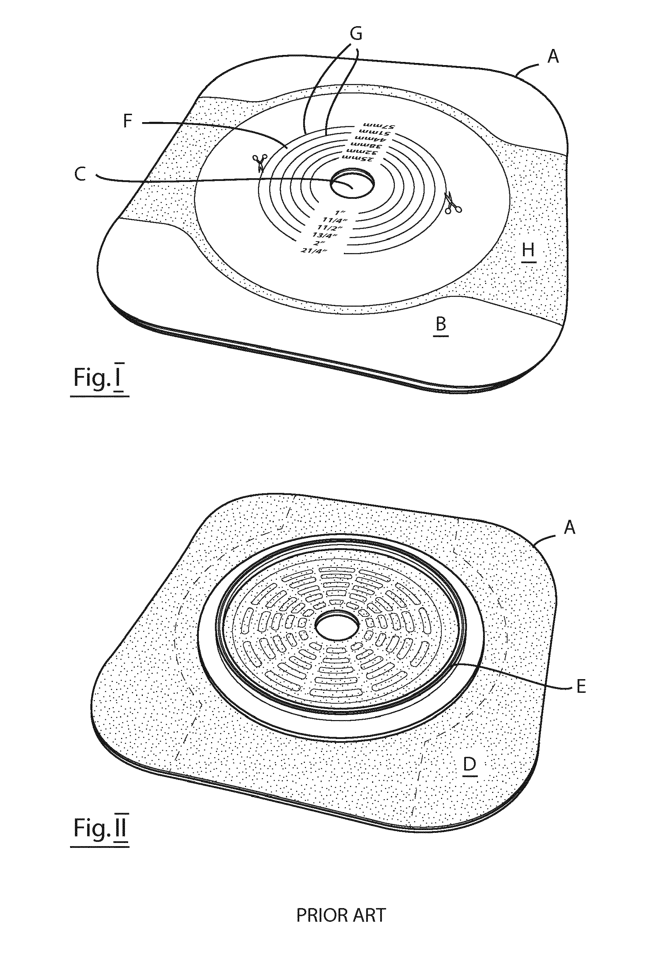

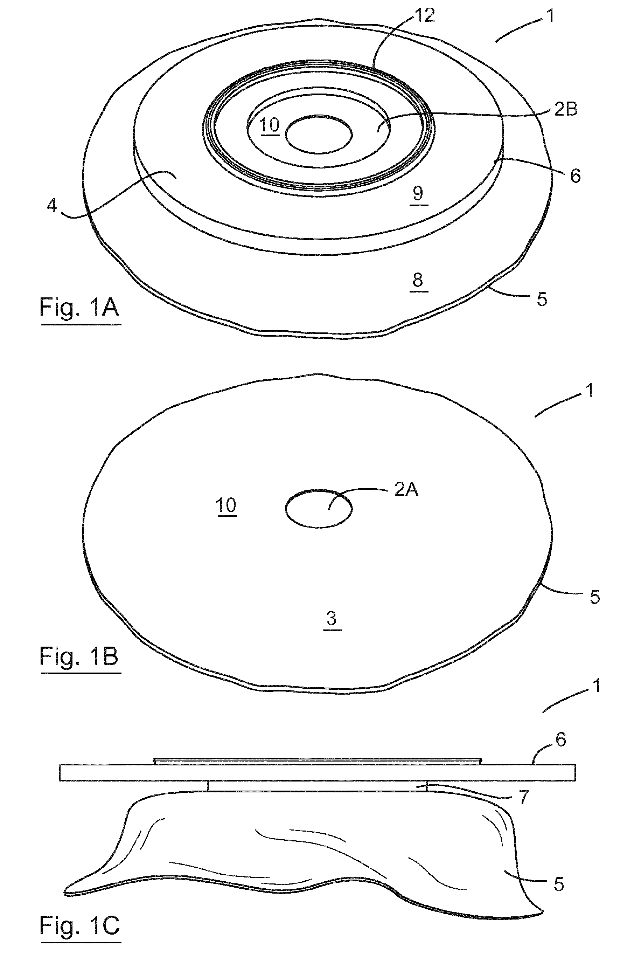

Referring to the Figures, and initially to Figs 1A to 1C, there is illustrated a fixing plate 1 for an ostomy pouching system according to a preferred embodiment of the invention. The plate 1 comprises a central hole, a first major surface 3 that in use abuts a patient's body, and a second major surface 4 that in use faces away from the patient's body. In more detail, the plate 1 is formed from two layers, a base layer 5 formed from a flexible silicone material and having a central hole 2A and an upper layer 6 formed from a less flexible, more rigid, silicone material having a polyurethane coating to aid adhesion, and having a larger central hole 2B, connected by means of an annular seal 7 surrounding the central holes 2A, 2B such that a peripheral part 8 of the base layer 5 and peripheral part 9 of the upper layer 6 are independent of each other (see Fig. 1C). The central part of the base layer 5 surrounding the central hole 2A comprises a thin layer of the flexible silicone material 10 (resiliently deformable region), whereas the peripheral parts 8 of the base layer comprise a thicker layer of the silicone material. The upper layer 6 also comprises a snap-fit attachment means 12 for engagement with a corresponding formation on an ostomy bag.

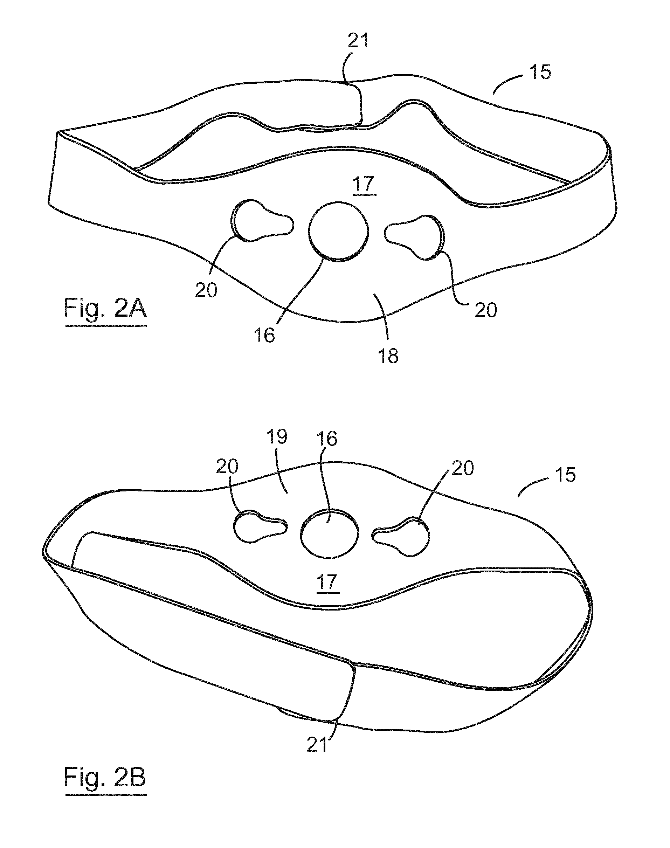

Referring to Figs 2A and 2B, and Fig. 7, there is illustrated means for attaching the fixing plate 1 to a patient's body comprising a belt 15 having a central hole 16 configured to receive the stoma and a surrounding surface 17 adapted to abut the fixing plate 1, the surrounding surface having a front face 18 (Fig. 2A) and rear face 19 (Fig. 2B). The belt 15 comprises further apertures 20, 20 located on each side of the central hole 16, which in use prevents the forces that are applied to the belt when tightening to be transmitted to the central hole (and thereby to the fixing plate, and provides for the forces to be channelled around the central hole (Fig. 7). The belt 15 also comprises a hook and loop fastening means 21 for attachment and tightening of the belt.

Referring to Figs. 3A and 3B, there is illustrated the fixing plate 1 and belt 15 in an assembled orientation with the upper layer 6 abutting the front face 18 of the surrounding surface 17 and the base layer 5 abutting the rear face 19 of the surrounding surface 17.

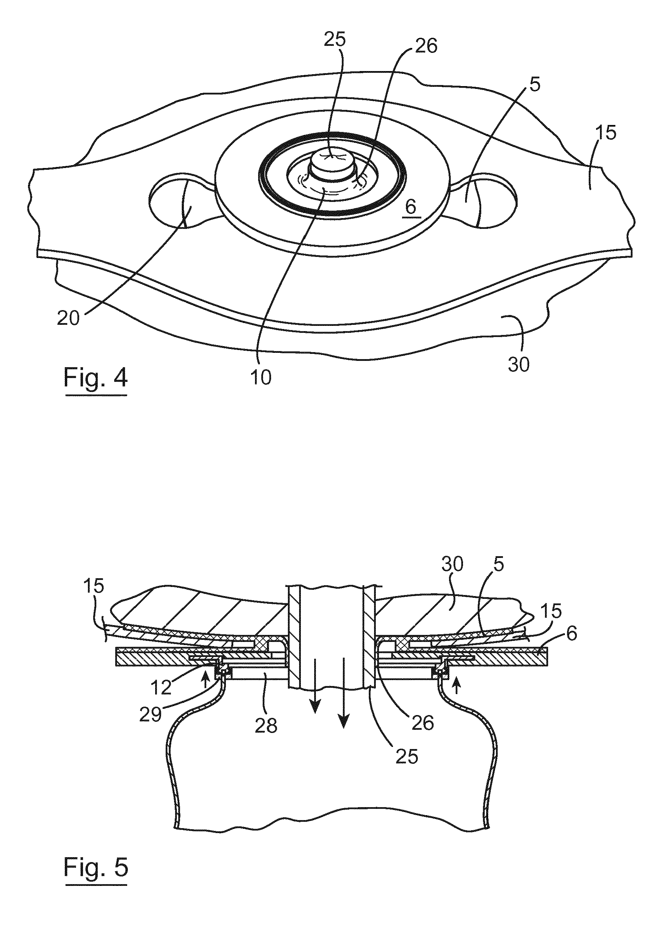

Figs 4 and 5 show the belt 15 and fixing plate 1 in-situ on a patient's body, and the stoma 25 projecting through the central hole 2A in the thin layer of flexible silicone material 10 which forms a neck region 26 that conforms to a sidewall of the stoma 25. Fig. 5 shows the base layer 5 conforming to the contours of the patients skin 30 surrounding the stoma, and the stoma 25 projecting through the central hole 2A in the thin layer of flexible silicone material 10 and through the larger central hole 2B in the upper layer 6. Fig. 5 also shows an ostomy bag 27 having an aperture 28 and an annular snap-fit attachment 29 in engagement with the corresponding attachment 12 on the upper plate 6.

In use, and referring to Figs 6A to 6D, the fixing plate 1 (without any bag attached) is assembled with the belt 15 by pulling the flexible base plate 5 through the central hole 16 in the belt (Fig. 6A). Once assembled, the base layer 5 abuts rear face 19 of the belt 15 and the upper layer 6 abuts the front face 18 of the belt, and the through holes 2A and 2B are aligned with the central hole 16 in the belt 15 (Fig. 6B). The belt and fixing plate assembly is then position against the patients skin with the aligned holes (2A, 2B and 16) aligned with the stoma 25, and the plate is pressed against the patients skin 30 such that the stoma 25 is pushed through the thin layer of flexible silicone material 10 forming part of the base layer 5, such that the silicone material forms a neck region 26 that conforms tightly to the sidewall of the stoma (Fig. 6C). Fig. 6D illustrates how the peripheral part 8 of the upper layer 6 can move independently of the peripheral part 9 of the base layer 5. Once in-situ, the belt 15 is tightened to secure the fixing plate in position, and a bag (not shown) is attached to the upper plate by means of the snap-fit attachment means.

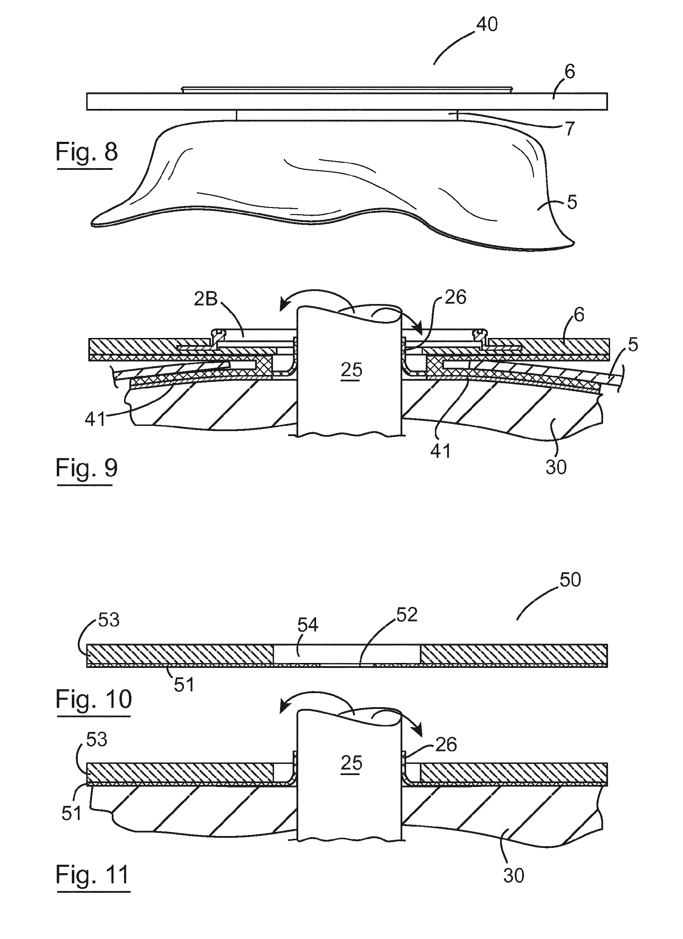

Referring to Figs 8 and 9, there is illustrated an alternative embodiment of the invention in which parts described previously with reference to Figs 1 and 2 are assigned the same reference numerals. In this embodiment, the fixing plate 40 is essentially the same as that described previously, although in this embodiment the base layer 5 is attached to the skin with a layer of adhesive 41. This embodiment of the invention addresses the problem of chemical dermatitis

The use of this embodiment is essentially the same as that described with reference to the previous embodiment, with the exception that the fixing plate is attached to the patient's skin by means of a layer of adhesive formed on the base layer as opposed to the use of an attachment belt.

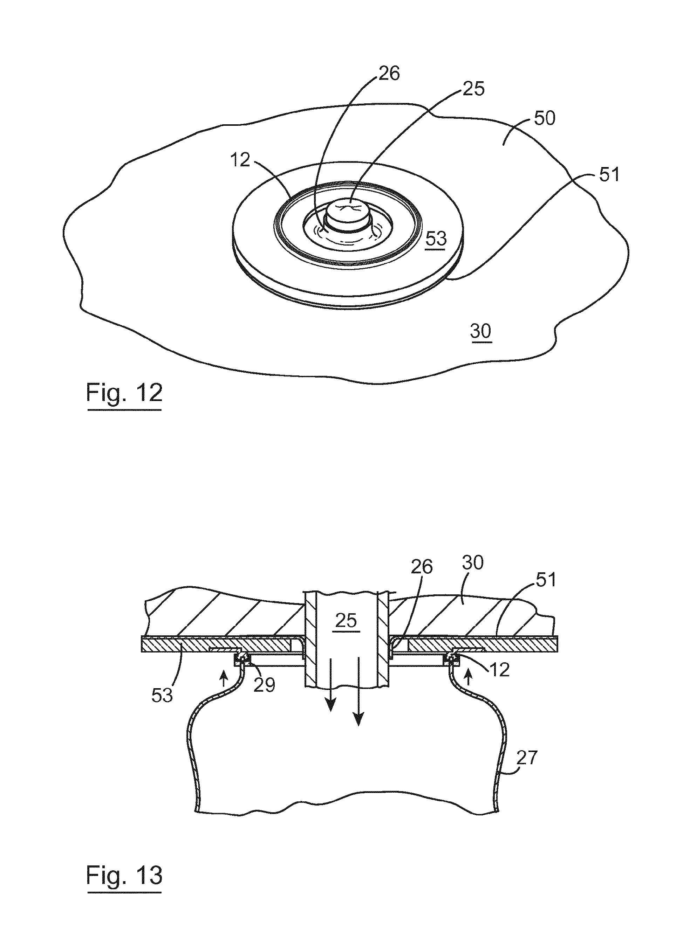

Referring to Figs. 10 and 11, there is illustrated a further embodiment of the invention, in which parts described previously with reference to the previous embodiments are assigned the same reference numerals. In this embodiment, the fixing plate 50 comprises a laminated structure having a base layer 51 of thin, flexible, silicone material with a small hole 52, and a top layer 53 formed of a polyurethane material with a central hole 54 that is larger than the hole 52. The central portion of the base layer 51 surrounding the hole 52 forms the resiliently deformable region 10. In use, and referring to Figs 11 to 13, the plate 50 is pressed over the stoma 25 in the manner previous described until the stoma protrudes through the hole 52 and the resiliently deformable region 10 forms a neck region 26 that conforms tightly to the sidewall of the stoma. An ostomy bag 27 is then attached to the upper layer 53 by means of snap-fit connectors (12, 29) formed on the bag and the upper layer.

Referring to Fig. 14, there is illustrated a further embodiment of the invention, in which parts described previously with reference to the previous embodiments are assigned the same reference numerals. In this embodiment, the plate 1 comprises a convex silicone base layer 5 having a central recessed portion 26 and a thin layer of the flexible silicone material that in use forms a neck portion on the stoma 25 when the stoma projects into the recessed portion 26. This embodiment is especially useful for patients having a short stoma or patients having a soft abdomen.

Referring to Fig. 15, there is illustrated a further embodiment of the invention, in which parts described previously with reference to the previous embodiments are assigned the same reference numerals. In this embodiment, the system comprises a fixing plate 1 that is integrally formed with the ostomy bag 27 having an aperture (dashed line 60). The fixing plate 1 is formed by a layer of silicone material having a thin layer of flexible silicone material 10 surrounding a central hole 2A, wherein the central hole and the thin layer of flexible silicone material 10 have a diameter that is smaller than the diameter of the aperture of the ostomy bag. Although not shown, a periphery of the fixing plate 1 surrounding the thin layer of flexible silicone material 10 may be coated with a layer of polyurethane or other similar material suitable for application or receipt of adhesive, in which the fixing plate is attached to the bag by means of adhesive applied to the periphery of the fixing plate.

### Chemical dermatitis solution

The base layer of the fixing plate, due to its shape and the flexible nature of the material selected, forms a skin protector by creating a flush seal around the base of the ostomy. This prevents ostomy output waste from coming into contact with the skin.

Base layer (skin protector) material can be silicone with a silicone adhesive backing. Silicone adhesive, due to its tackiness prevents migration of the skin protector from around the area of the stoma.

### Mechanical dermatitis solution

A belt with a fastening mechanism, whereby the belt houses the fixing plate which connects to the bag. As such adhesive may not be required for bag attachment- potentially avoiding mechanical dermatitis.

A skin protector with a bag attachment mechanism: The bag may be directly attached to the skin protector material. If the skin protector is adhered directly on to the skin, then the bag can be secured directly to the skin protector if the garment is not being used. Due to the nature of the novel material selected, it is less likely to cause mechanical dermatitis as described in "Advantages". It can also remain on the body whilst the bag is being removed/changed, so repeated removal of adhesive from skin is not required.

### Lack of Security Solution:

A belt with a connection method that is not directly on the ostomate's skin.

Method of attaching the bag to the belt rather than the ostomate's skin allows a secure connection without the necessity of having a flush surface to connect with the bag.

The bag is secured to the protector rather than the ostomate's skin, the protector is housed in the garment and the garment is anchored to the ostomate's body.

Ostomy output waste can only flow around the skin protector and into the bag avoiding all contact of waste with the skin. This along with the new attachment of the bag to the belt will decrease the risk of leaks, thus giving confidence to the ostomate.

In another embodiment the silicone adheres to the body without the use of the belt.

The skin protector acts as a locator for device positioning due to its visual and tactile design. A more rigid upper layer provides a more stable surface to which the bag can be attached, enabling enhanced security.

### Advantages of Garment

### Helps prevent/manage skin irritancy.

No other garment has been identified where the ostomy bag can be attached directly onto the garment. By attaching directly on to the garment, the device avoids the risk of causing mechanical dermatitis as nothing will be stuck directly onto the skin. Furthermore if a person with an ostomy is suffering from skin complications, then the garment is an appealing option as it means attaching a bag or a wafer directly onto irritated skin can be avoided.

### Offers enhanced attachment security

Currently, wearers of ostomy bags are often concerned that their bag is not attached securely. With the advent of the current garment, bags can be attached more securely, offering wearers increased confidence during daily activities.

### Potential to conceal bag

In cases when people with ostomy bags do not want the bag to be visible, it will be possible to conceal the bag in the garment, offering wearers discretion.

### Additional advantages

Anti-odour material (bamboo carbon or other as a selected material) will help in masking smells of gas passing through the ostomy

The garment can, with appropriate design offer orthopaedic support, particularly for the lumbar region.

The garment may help in offering an external support to either manage or prevent peristomal hernias.

Thermal regulation (through the selection of an appropriate material; breathable materials) Antimicrobial: The garment could have an antibacterial and antifungal additive that may reduce infection and odour hazards.

The garment can be low profile and discreet.

The garment can be flexible, resulting in an easy fit for a broad range of sizes.

### Advantages of the Skin Protector

### Prevents waste from contacting the skin.

Because of the design of the skin protector, it can wrap around the protruding ostomy to prevent the possibility of any waste flowing backwards onto the skin from the stoma exit. As such, there will be little, if any risk of causing chemical dermatitis.

### Limits migration

By combining with the garment, the device will offer a secure attachment. The material selected (silicone or other) to be in contact with the skin can be conditioned to have a tacky/sticky surface. As such, it will increase security in the bag's attachment to the ostomy as well as preventing migration.

### Reduces risk of mechanical dermatitis

The skin protector in the current design does not have to be removed every time a bag is replaced or taken off. As such, mechanical dermatitis caused by repeatedly removing the bag will be reduced. Furthermore, an alternative material to current adhesives will be used to secure the skin protector to the skin.

### No shaving or prep of the peristomal skin required.

As the skin protector can be attached and secured without the use of adhesive, shaving and cleaning of the skin. If the patient uses creams on their skin, this will not interfere with the attachment of the bag, unlike existing products.

### Facilitate in bag positioning

As the skin protector is put on before the bag, it can be put on by the ostomate with confidence that they have placed it securely and safely around the stoma. This should help in reducing the risk of waste contact with the skin.

### Therapeutic benefits of novel materials.

The repeated removal of standard ostomy bags, results in the peeling off of the outer layers of the epidermis- due to its sticking to the current adhesives. This "skin stripping" alters the barrier properties of the skin and exposes deeper skin cells, leaving the area more susceptible to attack from effluent, bacteria and from the adhesive ingredients. The rate of transepidermal water loss also increases. The use of a silicone gel has been shown to compensate to the absence of outer layers of the epidermis by acting as a water barrier. As such, the use of silicone gel at the interface between the skin protector and the skin would act as a water barrier, compensating for the absence of the outer layers of the epidermis, caused by repeated removal of ostomy bags and ultimately resulting in mechanical dermatitis. It is proposed the materials used in the current device will have a therapeutic effect, helping to manage complications associated with mechanical dermatitis.

### Advantages of the Bag Attachment

In one embodiment, the current device will have a mechanical attachment method for securing the ostomy bag to the person. In this embodiment, this anchor system will be part of/attached to the garment. This attachment method will:
Offer security in how the bag attaches to the ostomy- a reduced risk of it coming off.
Prevent the backflow of any waste onto the skin. The design will insure a secure connection between the ostomy bag and the interface. Waste will flow directly into the ostomy bag and no gaps or leaks will exist, resulting in less risk of chemical dermatitis.

In a second embodiment, the mechanical mechanism for attaching the ostomy bag will be embedded into the skin protector, in that the skin protector will have an attachment mechanism to which a bag can be mechanically secured. Like the first embodiment, this attachment will:
Offer security in how the bag attaches to the ostomy- a reduced risk of it coming off
Prevent the backflow of any waste onto the skin. The design will insure a secure connection between the ostomy bag and the interface. Waste will flow directly into the ostomy bag and no gaps or leaks will exist, resulting in less risk of chemical dermatitis.

In another embodiment, ostomy bags can be adhered to either the skin protector or to part of the garment. As in the first and second embodiments, this will:
Offer security in how the bag attaches to the ostomy- a reduced risk of it coming off
Prevent the backflow of any waste onto the skin. The design will insure a secure connection between the ostomy bag and the interface. Waste will flow directly into the ostomy bag and no gaps or leaks will exist, resulting in less risk of chemical dermatitis.

In another embodiment, the ostomy bag would attach to the garment by means of a hook and loop fastening system.

In all embodiments, the flow of waste from the stoma to the ostomy can occur through a passage that is completely sealed. That is,
at the entrance to the ostomy bag, no waste may leak out between the bag and the attachment. at the stoma exit, no waste may leak out onto the skin- underneath the skin protector.

### Additional material advantages

All materials can include anti odour properties by, for example, the addition of carbon, scented microspheres or other.

All materials can include antibacterial and anti-fungal properties by, for example, the addition of antimicrobial powders, silver or other.

Materials can be coloured/pigmented to offer discretion or to be fashioned as is considered cosmetically pleasing.

The invention is not limited to the embodiments hereinbefore described which may be varied in construction, detail and process step without departing from the spirit of the invention.

## Claims

1. An ostomy pouching system comprising a fixing plate and a bag having an opening, the fixing plate comprising: a central hole for receipt of a stoma; a first major surface that in use faces a patient's body; and a second major surface that in use faces away from the patient's body;, **characterised in that** the central hole has a perimeter that is smaller than a perimeter of the stoma, and at least a portion of the first major surface that surrounds the central hole comprises a resiliently deformable region capable of stretching to form a neck region that in use conforms to a sidewall of the stoma when the stoma protrudes through the central hole.

2. An ostomy pouching system as claimed in any preceding Claim in which the fixing plate comprises a layered structure having at least a base layer bearing the first major surface and formed of a flexible tacky material and an upper layer bearing the second major surface and formed of a material that is less flexible than the base layer.

3. An ostomy pouching system as claimed in Claim 2 in which the base layer comprises an annular peripheral region surrounding the resiliently deformable region that has a thickness greater than the thickness of the resiliently deformable region.

4. An ostomy pouching system as claimed in which the base layer is formed of a silicone material.

5. An ostomy pouching system as claimed in Claim 3 or 4 in which the base layer and upper layer are connected by an annular seal that surrounds the central hole such that an annular peripheral portions of the base layer and upper layer are not connected.

6. An ostomy pouching system as claimed in any preceding Claim including means for attaching the fixing plate to the patient's body, that optionally comprises belt means having a central hole configured to receive the stoma and a surrounding surface adapted to abut the fixing plate.

7. An ostomy pouching system as claimed in any of Claim 6 when dependent on Claim 5 in which the fixing plate is attached to the belt with the base layer disposed against one face of the belt and the upper layer disposed against an opposite face of the belt.

8. An ostomy pouching system as claimed in any of Claims 1 to 5 in which the means for attaching the fixing plate to the patient's body comprises adhesive means disposed on the first major surface of the fixing plate.

9. An ostomy pouching system as claimed in any preceding Claim in which the means for attaching the fixing plate to the bag comprises adhesive means or non-adhesive engagement means formed on the second major surface of the fixing plate or the bag or both.

10. A fixing plate forming part of an ostomy pouching system of Claim 1, and comprising: a plate member having a central hole for receipt of a stoma; a first major surface that in use faces a patient's body; a second major surface that in use faces away from the patient's body; optionally means for attaching the fixing plate to a patient's body; and optionally means for attaching the opening of the bag to the second major surface of the fixing plate surrounding the central hole, **characterised in that** the central hole has a perimeter that is smaller than a perimeter of the stoma, and at least a portion of the first major surface that surrounds the central hole comprises a resiliently deformable region capable of stretching to form a neck region that in use conforms to a sidewall of the stoma when the stoma protrudes through the central hole.

11. A fixing plate as claimed in Claim 10 in which the fixing plate comprises a layered structure having at least a base layer bearing the first major surface and formed of a flexible tacky material and an upper layer bearing the second major surface and formed of a material that is less flexible than the base layer.

12. A fixing plate as claimed in Claim 11 in which the base layer and upper layer are connected by an annular seal that surrounds the central hole such that an annular peripheral portions of the base layer and upper layer are not connected.

13. A fixing plate as claimed in Claim 11 in which the base layer is formed of a silicone material and the upper plate is formed of a material that is optimised for receipt of an adhesive layer or non-adhesive bag attachment means.

14. A method of attaching an ostomy pouching system according to any of Claims 1 to 9 to a patient having an ostomy, the method comprising the steps of: positioning the fixing plate against the body such that the stoma aligns with the central hole, pressing the fixing plate against the body such that the stoma is forced through the central hole and the resiliently deformable region of the first major surface stretches to form a neck region that conforms to a sidewall of the stoma, attaching the fixing plate to the patient's body, and attaching the opening of the bag to the second major surface of the fixing plate.

15. A method of attaching an ostomy pouching system according to Claim 7 to a patient having an ostomy, the method comprising the steps of: attaching the fixing plate to the belt such that the base layer of the fixing plate is disposed against one face of the belt and the upper layer of the fixing plate is disposed against an opposite face of the belt, positioning the fixing plate and the belt against the body such that the stoma aligns with the central hole, pressing the fixing plate against the body such that the stoma is forced through the central hole and the resiliently deformable region of the first major surface stretches to form a neck region that conforms to a sidewall of the stoma, attaching the fixing plate to the patient's body using the belt, and attaching the opening of the bag to the second major surface of the fixing plate.
